# EUROPEAN PATENT SPECIFICATION

(11) **EP 4 003 005 B1**
(45) Date of publication and mention of the grant of the patent: **02.08.2023**
(21) Application number: 20750361.6
(22) Date of filing: 17.07.2020
(51) Int. Cl.: A01N 1/02, A61F 2/06, A61B 17/00

(54) **CONDITIONING DEVICE FOR CONDITIONING AN EXPOSED STRETCH OF A BLOOD VESSEL**
KONDITIONIERVORRICHTUNG ZUM KONDITIONIEREN EINES EXPONIERTEN ABSCHNITTS EINES BLUTGEFÄSSES
DISPOSITIF DE CONDITIONNEMENT PERMETTANT DE CONDITIONNER UNE SECTION EXPOSÉE D'UN VAISSEAU SANGUIN

(30) Priority: 22.07.2019 IT 201900012537
(43) Date of publication of application: 01.06.2022
(73) Proprietor: Politecnico di Milano, 20133 Milano (IT); Centro Cardiologico Mozino S.p.A., 20121 Milano (IT); UNIVERSITA' DEGLI STUDI DI MILANO, 20122 Milano (IT)
(72) Inventor: FIORE, Gianfranco Beniamino, 20133 Milano (IT); SONCINI, Monica, 20133 Milano (IT); PIOLA, Marco, 20133 Milano (IT); PESCE, Maurizio, 20121 Milano (IT); AGRIFOGLIO, Marco, 20122 Milano (IT)
(74) Representative: Crippa, Paolo Ernesto
(86) International application number: PCT/IB2020/056750
(87) International publication number: WO 2021/014312

(56) References cited:
- WO-A2-98/20027
- US-B2- 9 579 224
- JOYCE CHEUNG-FLYNN ET AL: "Limiting Injury During Saphenous Vein Graft Preparation For Coronary Arterial Bypass Prevents Metabolic Decompensation", SCIENTIFIC REPORTS, vol. 7, no. 14179, 27 October 2017 (2017-10-27), XP055686699, DOI: 10.1038/s41598-017-13819-w
- PETER LAMM ET AL: "Continuous graft perfusion: Optimizing the quality of saphenous vein grafts", HEART SURGERY FORUM, vol. 5, no. Suppl 4, 1 February 2002 (2002-02-01), pages S355-S361, XP055687018,

## Description

### . Field of the invention

**.** The present invention relates to a conditioning device.

**.** A conditioning device according to the present invention is adapted to condition an exposed stretch of a blood vessel.

**.** A conditioning device according to the present invention is applicable to condition an exposed stretch of saphenous vein for a surgical procedure to collect material for autologous grafts, by way of non-limiting example for coronary bypasses.

**.**

### . Prior art

**.** Surgical procedures for the revascularization of cardiac tissues are generally known which provide exposing a portion of a patient's saphenous vein and severing a stretch for autologous grafts, e.g. coronary or vascular bypasses. The saphenous vein extends in a superficial portion of the leg between the venous arch of the foot and the groin, passing through the area of the medial malleolus, the knee, and the thigh.

**.** Typically, these surgical procedures provide making a longitudinal incision substantially on the entire medial side of the patient's leg to generate an elongated slit through which a predominant portion of saphenous vein is extracted and exposed, after having severed it near the malleolus forming a free end of the saphenous vein. In this manner, a surgical team is in a position to temporarily extract a predominant stretch of saphenous vein through said elongated slit, thus exposing it.

**.** By virtue of the stretch of exposed saphenous vein, the surgical team can collect biological material intended to form the substrate for an autologous graft, e.g. for applications such as coronary and/or vascular bypass.

**.** After collecting the biological material, usually a section of saphenous vein necessary for the operation (several centimeters in length according to surgical needs), the saphenous stumps are sutured to the malleolus and leg or thigh (according to the length of the harvested stretch) and the surgical closure of the wound is performed in layers.

**.** Typically, this consolidated surgical procedure requires the exposure of a predominant saphenous vein stretch for an interval of time during which the exposed saphenous vein stretch may experience undesirable phenomena due to the temporary interruption of physiological equilibrium, which leads to degradation of the features of the graft material, potentially limiting the service life of the graft. For example, exposure of the saphenous vein imposes oxidative stress on the exposed segment of the vein, which causes a dysfunction of the endothelial tissue forming the vein wall, which can degenerate into a remodeling of the vein walls as well as chronic inflammatory phenomena.

**.** In a time span of even a several years, such remodeling of the walls can result in the formation of a new intimate layer (neointima) and the formation of atherosclerotic plaques which reduce the patency of the graft early, causing an early failure, and imposing the implantation of stents in the graft or its replacement by a new surgical operation.

**.** The need is strongly felt to prevent the onset of mechanisms, which lead to the unwanted remodeling of the vessel wall, minimizing the damage due to the harvesting procedure of the vessel.

**.** To limit the damage due to the vessel harvesting procedure, some palliative remedies have been introduced in surgical practice, e.g. such as the application of gauze soaked in saline solution, to keep the exposed stretch of saphenous vein moist, thus avoiding dehydration.

**.** For example, it is also known from document US-9579224 a solution adapted to place a stent about a section of saphenous vein in situ to stimulate an inflammatory reaction to collect inflammatory cells in the area adjacent to the stent.

**.** Conversely, in the technical domain of vascular grafting, it is known, for example, from document WO-98-20027 to create a device for administering active ingredients on the outside of a vascular graft, wherein the device substantially consists of an impermeable cap fitted to the portion of the vessel where the graft is made and fixed thereto surround the vascular graft by means of an impermeable closing system which forms a sealing chamber between the outer walls of the vessel branches comprising the graft. A solution containing vascular endothelial growth factor (VEGF) is then injected into such sealed chamber to promote the tissue vascularization in the zone of the graft.

**.** Although for clinical applications other than the collection of autologous grafts, such a device would not solve the problem because it implies a local alteration of the physiological balance, imposing stress on the vessel wall applied by the impermeable closing system, e.g. comprising elastic bands which press on one or more sections of the vessel itself. This stress does not prevent the onset of mechanisms which lead to the unwanted remodeling of the vessel walls and therefore such a solution is unsuitable for the collection of material for autologous grafts on the saphenous vein.

**.** In contrast, it is generally known to reduce the risk of proliferation of viral and bacterial infections in wounds by applying patches made of selectively permeable materials, and in particular materials non-permeable to viruses and bacteria, to form a barrier which limits the risk of contamination.

**.** Patches of this type are made to adhere tightly against the skin around the wound to be treated and typically comprise a layer of hydrogel intended to contact the tissue inside the wound to be treated by promoting the collection of wound exudates to be collected in a container through the body of the hydrogel by providing a fluid suction duct connected at one end to the wound to be treated and leading to the opposite end in the exudate collection container. A negative pressure generator is usually associated with the suction line. Solutions of the type described above are disclosed, for example, in document WO-2004-37334**.**

**.** However, these solutions are not applicable in the procedure of collecting grafts from an exposed stretch of blood vessel, e.g. such as the saphenous vein, because they require the presence of a suction system for exudates of the wound.

**.** Therefore, the need to provide a solution capable of avoiding or at least minimizing the onset of undesirable phenomena due to the interruption, albeit temporary, of physiological equilibrium which leads to a degradation of the features of the material for grafts, to extend the service life of the graft itself.

**.** At the same time, the need is felt to maintain the conditions of physiological balance in an exposed section of a blood vessel intended for the collection of autologous grafts.

### . Solution

**.** It is an object of the present invention to solve the described drawbacks of the prior art and to provide a solution to the needs mentioned above.

**.** These and other objects are achieved by a device according to claim **1.**

**.** Some advantageous embodiments are the object of the dependent claims.

**.** According to an aspect of the invention, a conditioning device for conditioning at least one portion of an exposed stretch of a blood vessel for collecting biological material for grafts, comprising an envelope and at least one conveying channel which extends entirely within the envelope; wherein said envelope comprises a first layer comprising a first surface intended to face said exposed stretch, and a second layer comprising a second surface, opposite to said first surface; and wherein said at least one conveying channel comprises at least one first aperture which leads out of the envelope, so that said at least one conveying channel can be supplied with working fluid through said at least one first aperture. Said first layer is permeable to at least one component of the working fluid, so that, when said working fluid is in said at least one conveying channel, said at least one component, permeating said first layer diffuses until it reaches said first surface, to apply a conditioning action on said exposed stretch of the blood vessel.

**.** By virtue of the suggested solutions, improved performance is provided in terms of conditioning an exposed stretch of a blood vessel, e.g. the saphenous vein during the collection of graft material.

**.** The suggested solutions provide improved control over the conditioning of an exposed stretch of a blood vessel, such as the saphenous vein during the collection of graft material.

### . Figures

**.** Further features and advantages of the device and of the method will appear from the following description of its preferred embodiments, by way of non-limiting examples, with reference to the accompanying figures, in which:

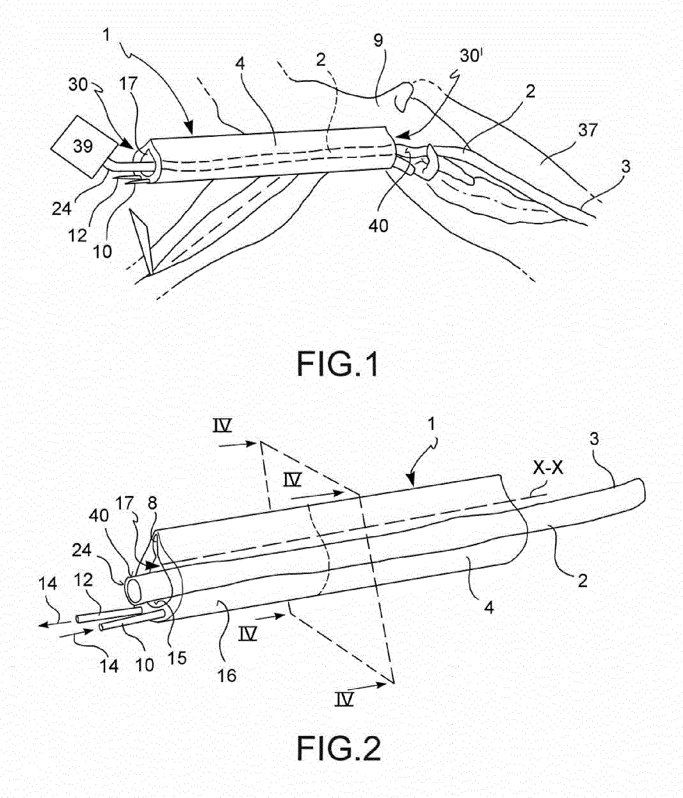

- figure 1 is a diagrammatic view of a step of the method of collecting, according to an operating mode;
- figure 2 is a diagrammatic axonometric view of a conditioning device according to an embodiment when in working conditions,

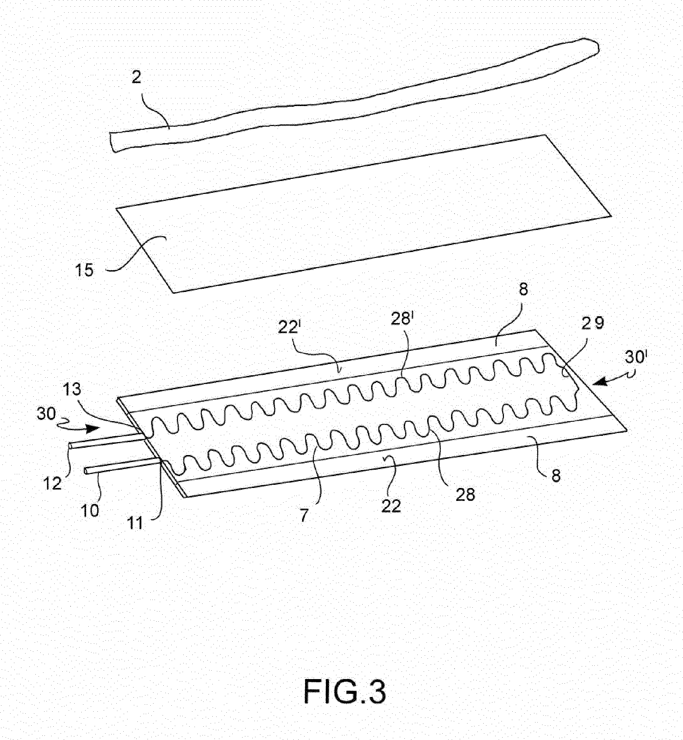

- figure 3 is an axonometric and separate parts view of the conditioning device in figure 2 in which an exposed stretch of a blood vessel is shown;
- figure 4-A is a view of a cross-section view of an elastic device of a conditioning device, according to an embodiment;
- figure 4-B is a section view taken along the plotting plane identified by arrows IV-IV-IV-IV in figure 2;
- figure 5-A is a view of a cross-section view of an elastic device of a conditioning device, according to an embodiment;
- figure 5-B is a section view similar to that in figure 4B which shows another embodiment,

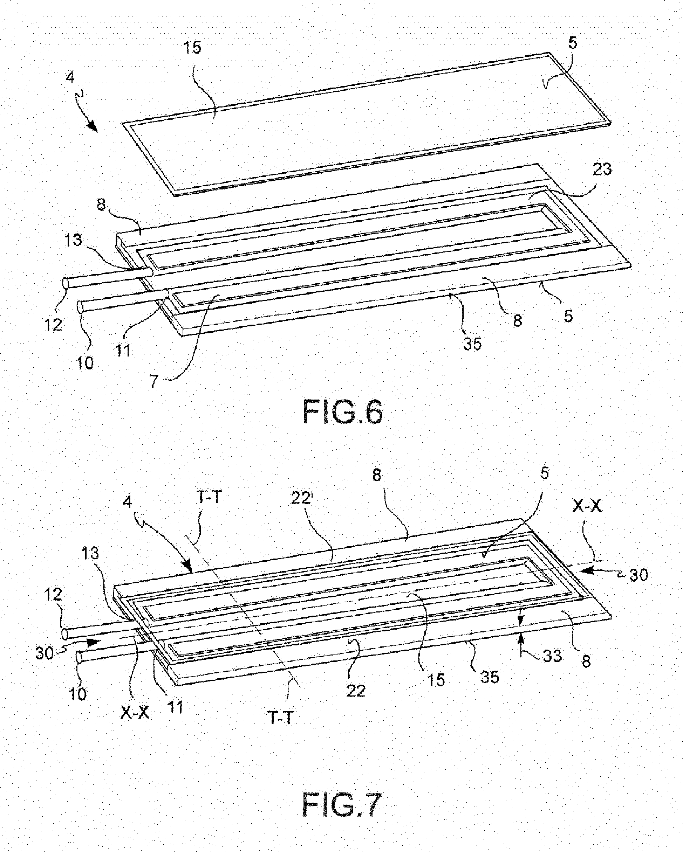

- figure 6 is an axonometric view with parts separated of a conditioning device, according to an embodiment.
- figure 7 is an axonometric which shows the conditioning device in figure 6;

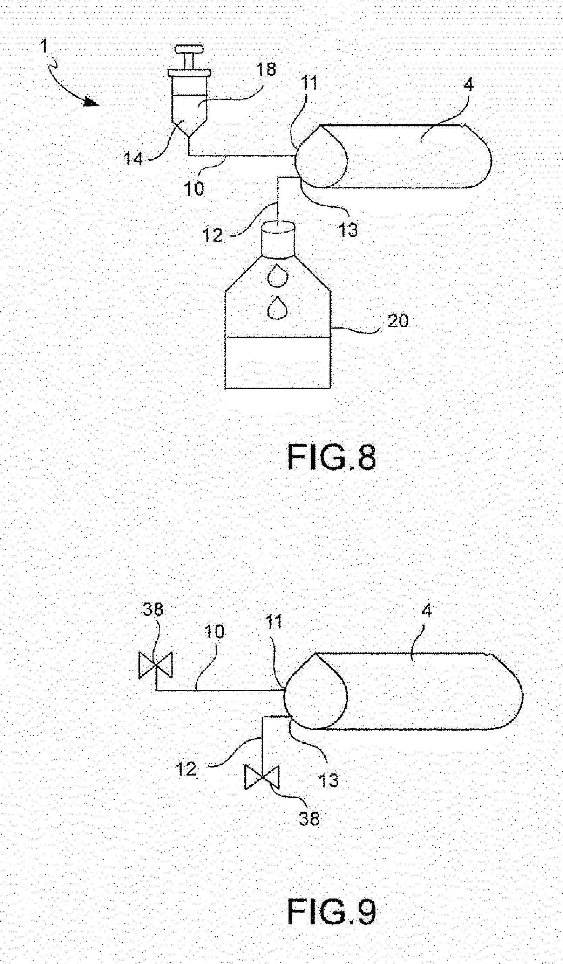

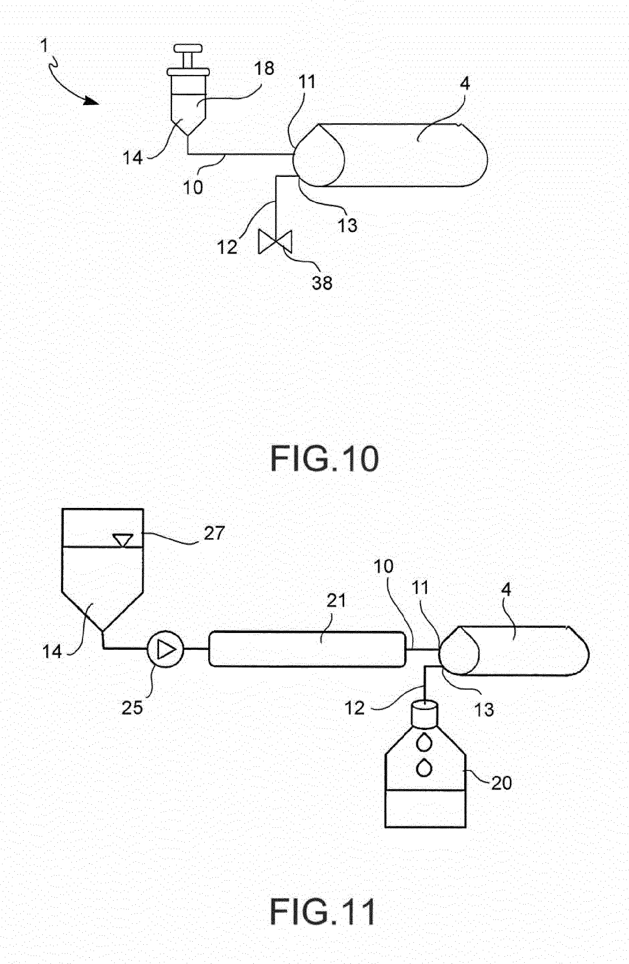

- figures 8, 9, 10 and 11 are diagrammatic views which show a conditioning device comprising a fluid supply device, according to some embodiments;

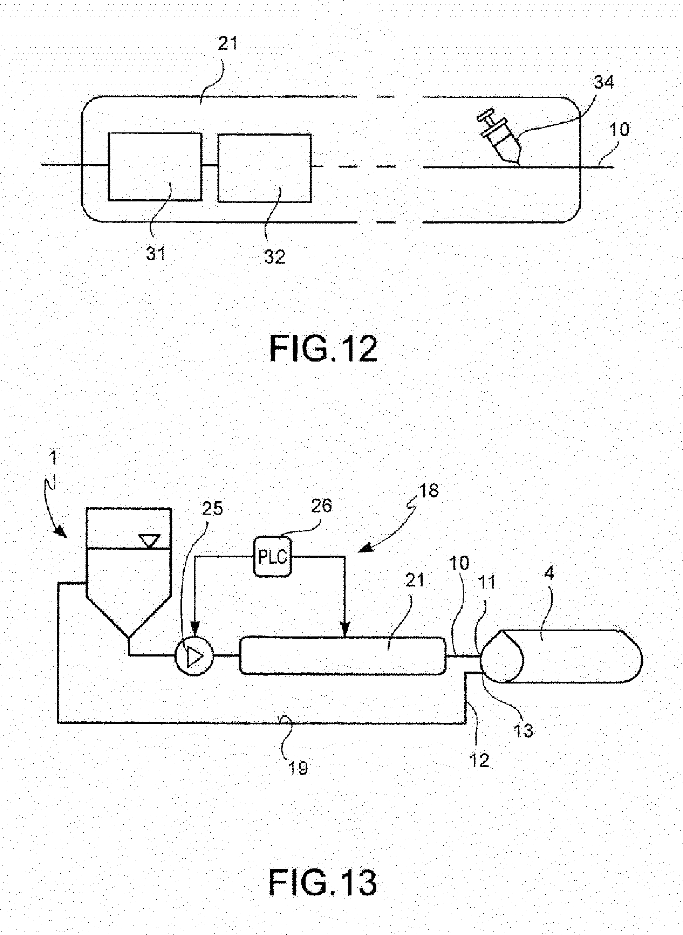

- figure 12 is a block chart showing an embodiment of a conditioning system of the working fluid of the conditioning device;
- figure 13 is a diagrammatic view of a conditioning device which comprises a recirculation path, according to an embodiment.

### . Description of some preferred embodiments

**.** According to a general embodiment, it is provided a conditioning device 1.

**.** Said conditioning device 1 is adapted to condition at least a portion of an exposed stretch 2 of a blood vessel 3.

**.** Preferably, said conditioning device 1 is used in the collection of biological material for grafts.

**.** Preferably, the term "exposed stretch" means a stretch of a blood vessel 3, which is temporarily taken outside the body of a patient.

**.** Preferably, the term "exposed stretch" means a stretch of a saphenous vein of a patient who, according to current surgical practice, is temporarily placed in paracorporeal position, with one end of the blood vessel stretch 3 still connected to the inside of the patient's body 37, and preferably the other end 24 severed and incannulated by a cannula 39 by an operator 9, typically a surgeon.

**.** Said conditioning device 1 comprises an envelope 4 or bag 4.

**.** Preferably, said envelope 4 of the conditioning device 1 comprises a casing 5 which at least partly delimits an inner volume 6. In this manner, said inner volume 6 is located inside said envelope 4 of the conditioning device 1. Preferably, said casing 5 encloses said inner volume 6. Preferably, said casing 5 is made of flexible material, e.g. in the form of film.

**.** Such conditioning device 1 comprises at least one conveying channel 7, or perfusion duct 7, extending entirely within said inner volume 6 of the envelope 4.

**.** Said envelope 4 comprises a first layer 15 comprising a first layer surface 15' or first surface 15' or first face 15' intended to face said exposed stretch 2.

**.** According to an embodiment, said first layer 15 and said first surface 15' are part of said casing 5, so that said casing 5 of the envelope 4 comprises said first layer 15.

**.** Said envelope 4 further comprises a second layer 16 comprising a second layer 16' or second surface 16' or second face 16', opposite to said first surface 15'.

**.** According to an embodiment, said second layer 16 and said second surface 16' are part of said casing 5, so that said casing 5 of the envelope 4 also comprises said second surface 16', opposite to said first surface 15' of the first layer 15 with respect to said inner volume 6.

**.** Said at least one conveying channel 7 comprises at least one first aperture 11 which leads out of the envelope 4, in other words, which leads out from the casing 5, so that said at least one conveying channel 7 can be supplied with working fluid 14 through said at least one first aperture 11. In this manner, said at least one first aperture 11 leads out from the inner volume 6.

**.** According to an embodiment, the conveying channel 7 comprises a single aperture 11 and acts as a working fluid collection vessel 14.

**.** Advantageously, said first layer 15 is permeable to at least one component 14' of the working fluid 14.

**.** In this manner, said working fluid 14, when there is in said at least one conveying channel 7, permeates with said at least one component 14' said first layer 15' of the envelope 4, so that said at least one component 14' reaches said first surface 15' of the envelope 4, to apply a conditioning action on said exposed stretch 2 of the blood vessel 3.

**.** In other words, when under operating conditions, said at least one component 14' of said working fluid 14, when said working fluid 14 is in said at least one conveying channel 7, spreads to said first surface 15' of the first layer 15 of the envelope 4, to apply a conditioning action on said exposed section 2 of the blood vessel 3.

**.** In this manner, it is possible, if necessary, to wet said first surface 15' by means of said first component 14'.

**.** Preferably, said first layer 15 forms a barrier for at least one additional component of said working fluid 14, except for said at least one component 14'. Advantageously, said first layer 15 is selectively permeable to at least one component 14' of the working fluid 14.

**.** Preferably, the term "conditioning action" means a homeostatic regulating action applied to the exposed stretch 2.

**.** Preferably, the term "conditioning action" means indicating an action to regulate environmental parameters, such as temperature and/or humidity and/or chemical composition.

**.** By virtue of the prevision of said conveying channel 7, it is possible to achieve a convective conveying of said working fluid 14 inside the conveying channel 7.

**.** By virtue of said first layer 15 permeable to said at least one component 14' of the working fluid 14, it is possible to make a diffusive, or mainly diffusive, or a slow convective conveying of said at least one component 14' of the working fluid 14 from the conveying channel 7 to the first surface 15'.

**.** Preferably, said second layer 16 is impermeable to said working fluid 14. According to a preferred embodiment, said second layer 16 forms at least one portion of an impermeable wall 23" of the conveying channel 7 which is impermeable to said working fluid 14.

**.** By virtue of the provision of said second impermeable layer 16, a barrier can be formed which prevents the transfer of the working fluid 14 and/or of each of its various components 14' to the outside of the envelope 4, i.e. of the casing 5. For example, the evaporation of the working fluid 14 is prevented. In this manner, a predetermined moisture level is maintained near the exposed stretch 2 of the blood vessel 3.

**.** By providing such a second layer 16, it is possible to improve the efficiency of said conditioning action.

**.** According to an embodiment, said conveying channel 7 is delimited at least partially by said first layer 15 permeable to said at least one component 14' of the working fluid 14. In this manner, at least a portion of wall 23 of the conveying channel 7 is made from said first layer 15. According to an embodiment, said inner volume 6 of the envelope 4 forms at least one portion of the wall 23' of the conveying channel 7.

**.** According to an embodiment, said inner volume 6 of the envelope 4 is in contact with said first layer 15.

**.** According to an embodiment, said first layer 15' allows said component 14' to permeate by capillarity towards said first surface 15'.

**.** According to an embodiment, said first layer 15' allows said component 14' to permeate by diffusion towards said first surface 15'.

**.** For example, said at least one component 14' of said working fluid 14 comprises at least one of either: water in liquid form, water vapor, dissolved oxygen, gaseous oxygen, bound oxygen, gaseous carbon dioxide, dissolved carbon dioxide, bound carbon dioxide, dissolved solutes, ionic solutes, bound solutes, non-soluble substances, gaseous substances, drug molecules bound to a dissolved carrier, drug molecules in suspended form, drug molecules in colloidal form, drug molecules in gaseous form, nano-particles, vesicles, combination of the above.

**.** For example, said working fluid 14 is a mixture and/or solution and/or suspension.

**.** For example, said working fluid 14 comprises, in addition to said component 14', at least one of either: water, saline solution, preferably in liquid form.

**.** For example, said working fluid 14 is in gaseous or aeriform form and comprises water vapor and/or a gas mixture.

**.** The construction of interconnected pores in the body of the inner volume 6 and/or the first layer 15 may be envisaged; such interconnected pores are preferably not to be considered as parts of the said conveying channel 7.

**.** By virtue of the provision of such a conditioning device 1, it is possible to transfer at least one component 14' of the working fluid 14 to the first surface 15' controlling the chemical-physical conditions thereof. In this manner, a portion with controlled chemical-physical conditions faces an exposed stretch 2 of the blood vessel 3 placed near or in contact with said first surface 15'.

**.** For example, said first layer 15 may allow the transfer of the aqueous component of working fluid 14 to the first surface 15', thus wetting it. In this manner, it overlooks an exposed stretch 2 of blood vessel 3 placed near or in contact with said first surface 15' a damp or moistened face of conditioning device 1. Therefore, the risk of dehydration of the exposed stretch 2 of the blood vessel 3, e.g. a saphenous vein, is avoided or at least minimized.

**.** Such working fluid 14 acts as a carrier element for conveying of conditioning factors in exposed stretch 2 of the vessel 3, e.g. the saphenous vein, such as humidity and/or temperature.

**.** Such a working fluid 14 may also act as a carrier element for the conveying of soluble and/or suspended chemical and/or biochemical components, for the conditioning of exposed section 2 of vessel 3, e.g. the saphenous vein.

**.** Preferably, when under exercise conditions, the first surface 15' and preferably the first layer 15 is in chemical balance with the exposed stretch 2 of the blood vessel 3. For example, the oxygen saturation value is substantially the same in the first surface 15' and in the wall 40 of the exposed section 2 of the blood vessel 3.

**.** According to a preferred embodiment, said at least one conveying channel 7 further comprises at least a second aperture 13 which leads out from said casing 5 of the envelope 4. In this manner, said at least one first aperture 11 and said at least one second aperture 13 leads out from the casing 5. Preferably, said at least one first aperture 11 acts as an inlet aperture and said at least one second aperture 13 act as an outlet aperture for the working fluid 14, so that a flow of working fluid 14 passes through said first aperture 11, said conveying channel 7, and said second aperture 13.

**.** By virtue of the provision of said inlet aperture 11 and of said outlet aperture 13, a convective flow of working fluid 14 through said inlet aperture 11 flows inside said envelope 4, flows into said conveying channel 7 and flows out from said outlet aperture 13 out from the envelope 4, in other words out from the inner volume 6 and/or out from the casing 5.

**.** The walls 23' which delimit said at least one conveying channel 7 may be made from said same inner volume 6 and may comprise porosity, being wettable, e.g. they can be adapted to absorb of at least one component of the working fluid 14 and/or a fraction of the working fluid flow.

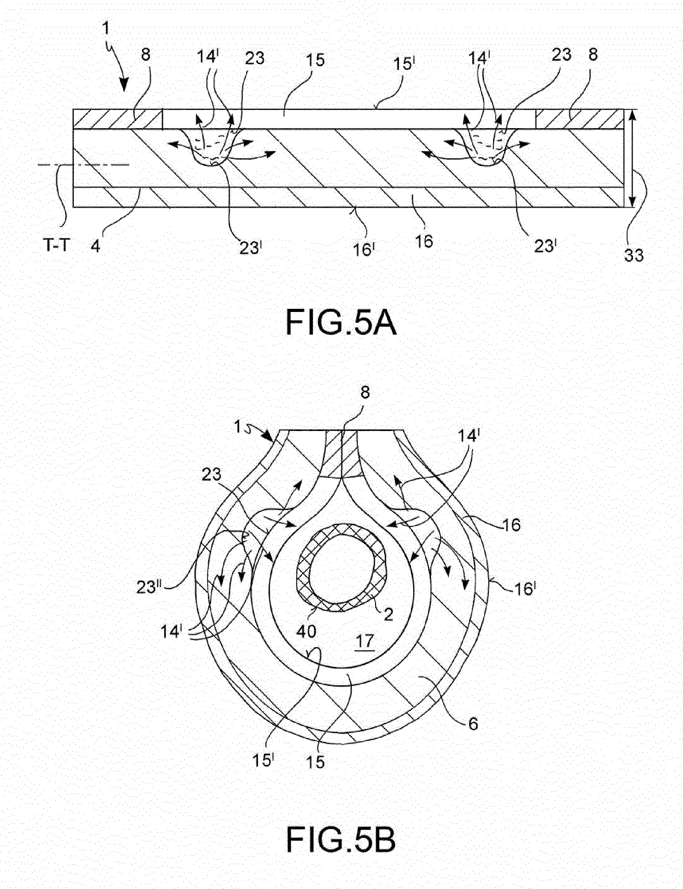

**.** In an embodiment shown for example in **figure 5****-A**, said inner volume 6 acts substantially as a third layer, sandwiched between said first layer 15 and said second layer 16, wherein said third layer formed by said inner volume 6 is permeable to said at least one component 14' of the working fluid 14. For example, said third layer formed by said inner volume 6 is made of silicone material and said first layer 15 is made of hydrogel. The provision of a third layer formed by said inner volume 6, and also having permeability for said at least one component 14', allows said at least one component 14' to diffuse on portions of said first surface 15' further away from the conveying channel 7. In other words, the prevision of at least one first wall 23 of the conveying channel 7 is formed by said first layer 15 and at least one second wall 23' of the conveying channel 7 is formed by said inner volume 6 permeable to said at least one component 14' allows the diffusion of the at least one component 14' through both said first and second walls 23, 23', distributing itself substantially over the entire first surface 15', preferably in a substantially uniform manner. The permeability of the inner volume 6 or third layer 6 said at least one component 14' of the working fluid 14 may be less, equal, or greater than that of the first layer 15. For example, the permeability of the inner volume 6 to said component 14' is intermediate to the permeability of the first layer 15 and the permeability of the second layer 16.

**.** According to an embodiment, said casing 5 is interrupted only at said at least one inlet aperture 11 and said at least one outlet aperture 13. In this manner, the casing 5 encloses and confines the inner volume 6 even if inner volume 6 is permeated by said working fluid 14 or component 14' thereof.

**.** According to an embodiment, said at least one conveying duct 7 is associated with at least one inlet duct 10 associated with said at least one first aperture 11 and at least one outlet duct 12 associated with said at least one outlet aperture 13, said at least one outlet duct 10 and said at least one outlet duct 12 extend outside said inner volume 6 of the envelope 4, preferably through said casing 5. Preferably, so-called ducts 10 and 12 are formed by flexible sleeves.

**.** According to an embodiment, said conveying channel 7 is made by digging into the second layer 16. For example, the second layer 16 is made of polymeric material, e.g. polyurethane and/or polyester.

**.** According to an embodiment, said conveying channel 7 is made by removing material from said second layer 16, on the side of the second layer 16 opposite to said second surface 16'. For example, said connecting channel 7 is made by laser etching.

**.** According to an embodiment, said conveying channel 7 is made by molding or casting. For example, the geometry of said conveying channel 7 is made in negative on a mold.

**.** According to an embodiment, said conveying channel 7 comprises at least one duct in which the working fluid 14 flows substantially in laminar flow.

**.** According to an embodiment, said conveying channel is made by hot embossing.

**.** Preferably, the first layer 15 and the second layer 16 are glued and/or welded together either directly or indirectly by overlapping at least one more layer, e.g. a third layer consisting of said inner volume 6.

**.** According to a preferred embodiment, said conditioning device 1 further comprises at least one locking device 8 or closing device 8, adapted to lock said envelope 4 at least temporarily in a predefined geometric configuration.

**.** Preferably, said predefined geometric configuration is a closed geometric configuration which forms a device lumen 17 adapted to receive said at least one exposed stretch 2 of the blood vessel 3. In this manner, it is possible to arrange said first surface 15' of said first layer 15 of the conditioning device 1 about the outer wall 40 of the exposed stretch 2 of the blood vessel 3.

**.** Preferably, said device lumen 17 forms a longitudinal cavity through which it passes. Said device lumen 17 may have a tapered shape or in any case be suitably shaped to adapt to accommodate the volumetric conformation of an exposed section 2 of a natural vessel.

**.** According to an embodiment, said device lumen 17 forms a longitudinal cavity adapted to be placed on or wrapped about the exposed stretch 2 of the blood vessel 3. In this manner, said envelope 4 substantially forms a sleeve to embrace said exposed stretch 2.

**.** According to a variant, device lumen 17 is not a passing lumen, i.e. it is a vessel comprising a bottom portion opposite the access aperture of the lumen 17, forming a cap for said exposed stretch 2.

**.** Preferably, in said envelope 4 a longitudinal direction X-X, substantially parallel or coincident with the longitudinal development axis of said exposed stretch 2 of blood vessel 3 is defined when said conditioning device 1 is in working condition, a transverse direction T-T, orthogonal to, and incident to longitudinal direction X-X, and a thickness direction, orthogonal to both longitudinal direction X-X and transverse direction T-T, and defining an envelope thickness 33. Preferably, said casing 5 defines said envelope thickness 33. Preferably, said envelope thickness 33 is much smaller than the extensions of said envelope 4 in longitudinal direction X-X and transverse direction T-T, so that said envelope 4 essentially forms a sheet.

**.** According to an embodiment, said at least one locking device 8 is placed on a transverse margin 22, 22' of said envelope 4. Preferably, the term "transverse margin" indicates a portion of said envelope 4 placed near or at the transverse edge of said envelope 4, wherein the longitudinal dimension of the envelope 4 is significantly larger, and preferably much larger, than the transverse extension of said envelope 4. Not necessarily said transverse margin 22, 22' is placed at edge 35 of envelope 4, although in accordance with a preferred embodiment it is.

**.** By virtue of said locking device 8, it is possible to keep said envelope 4 of conditioning device 1 around the exposed stretch 2 in wound configuration, like a sleeve.

**.** The joint provision of said second impermeable layer 16 and said locking device 8 allows, by wrapping said envelope 4 of the conditioning device 1 about the exposed stretch 2 of blood vessel 3, to form a containment barrier which helps to maintain the desired chemical-physical conditions in the device lumen 17, and therefore in the exposed stretch 2 of blood vessel 3 received in the device lumen 17 and facing said first surface 15', allowing an improved control over the conditioning action applied by the conditioning device 1 on the exposed stretch 2 of blood vessel 3.

**.** When in closed and wound configuration, the second layer 16 forms an impermeable barrier to working fluid 14 for conditioning the exposed section 2.

**.** According to a preferred embodiment, the inner volume 6 of said envelope 4 of the conditioning device 1, as well as said envelope 4, is flexible at least in a transverse direction, transverse to the longitudinal direction, so that it can be wound, in other words wrapped, about the exposed stretch 2 to embrace the exposed stretch 2 of blood vessel 3, e.g. the saphenous vein. The provision of the locking device 8 allows the envelope 4 to be locked in wrapped configuration about the exposed stretch 2 of blood vessel 3.

**.** Preferably, said envelope 4 comprises a locking device 8 on each transverse margin 22, 22', so that the envelope 4 can be wrapped to embrace said exposed stretch 2 of blood vessel 3 and locked in this geometric configuration, at least when under operating conditions, e.g. during collecting operation of the grafts.

**.** According to an embodiment, said at least one locking device 8 forms a releasable coupling. In this manner, the envelope 4 can be reopened if necessary, e.g. when the graft collection operation has ceased.

**.** According to an embodiment, said locking device 8 is adapted to form a slot-hook type coupling. Preferably, said at least one locking device 8 comprises Velcro.

**.** According to an embodiment, said locking device comprises a snap coupling system.

**.** According to an embodiment, called at least one locking device 8 is adapted to form an adhesive bond and is preferably made at least partially of adhesive material. Not necessarily said at least one locking device 8 comprises glue, although according to an embodiment which comprises it. According to an embodiment, said locking device 8 is adapted to form an adhesive bond by adjusting the surface features, such as the choice of the material of clamping device 8, the surface affinity of a material to the same material or a different material.

**.** According to an embodiment, said at least one locking device 8 is adapted to form a magnetic coupling. Preferably, said at least one locking device 8 comprises a permanent magnet. According to an embodiment, said at least one locking device 8 comprises at least one electromagnetic which can be activated as required.

**.** When in operating conditions in which the envelope 4 assumes a closed geometric configuration and wrapped around the exposed stretch 2 of the blood vessel 3, the device lumen 17 is oriented substantially in the X-X longitudinal direction.

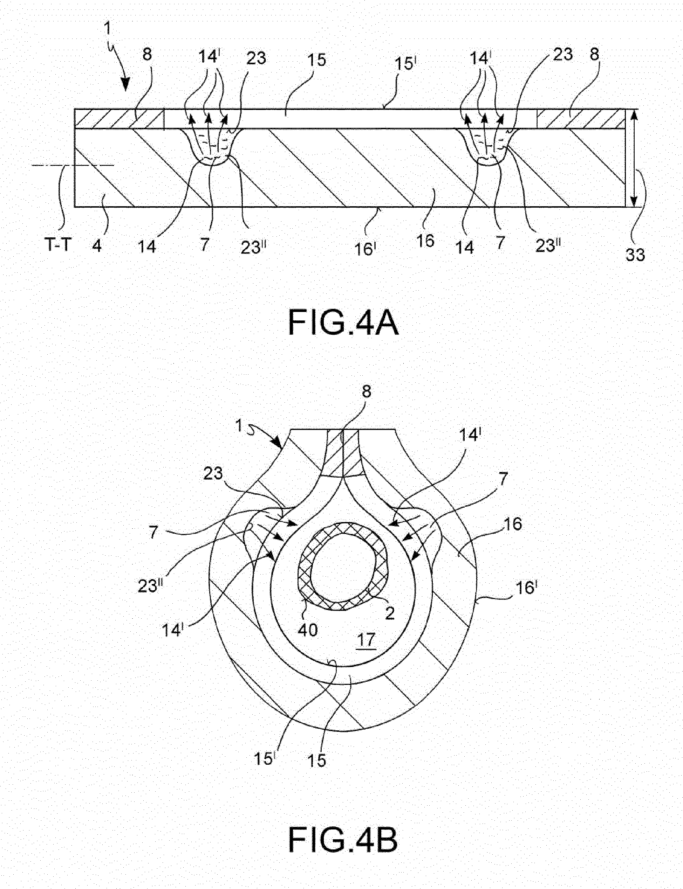

**.** By virtue of the locking device 8, the envelope 4 can switch from an open configuration, as diagrammatically shown in **figure 4****-A**, to a closed and wound configuration, as diagrammatically shown in **figure 4****-B**, wherein, when in wound configuration, the transverse dimension 36 of the envelope is reduced compared to when in an open configuration, while instead, the longitudinal extension of envelope 4 remains substantially constant.

**.** The provision of such a locking device 8 allows a quick locking of the envelope 4 in closed and wound configuration around the exposed stretch 2 of the blood vessel 3 while providing a firm locking of the envelope 4 in closed and wound configuration.

**.** According to an embodiment, said conveying channel 7 defines at least one coil-like perfusion path 28, 28' to maximize the exchange area of the internal volume 6 of the envelope 4 with the conveying channel 7. According to an embodiment, said conveying channel 7 defines at least two opposed coil-like paths 28, 28' which extend along the extension in longitudinal direction X-X of the envelope 4 and are joined together by a union duct 29, preferably placed on a longitudinal side 30 or 30' of the body of the envelope 4.

**.** According to an embodiment, said conveying channel 7 defines stagnation zones, e.g. having increased cross-section, to favor the diffusion of said at least one component 14' towards said first surface 15' of the device 1 and thus towards said exposed stretch 2 of blood vessel 3. According to an embodiment, said conveying channel 7 defines shuffling zones, e.g. forming an increased tortuosity, to obtain a mixing effect of said working fluid 14.

**.** According to an embodiment, said conveying duct 7 substantially extends along the entire longitudinal extension of the envelope 4.

**.** According to an embodiment, said conveying channel 7 defines at least one bifurcation. For example, at least one bifurcation is located just downstream of inlet duct 10.

**.** According to an embodiment, said inlet aperture 11 and said outlet aperture 13 of the conveying channel 7 are placed on the same longitudinal side 30 or 30' of the envelope 4, and preferably are both placed on the longitudinal side 30 of the envelope 4 adapted to face the free end 24 of the exposed stretch 2 of the blood vessel 3, e.g. the saphenous vein 3, when in operating conditions.

**.** According to a preferred embodiment, said first layer 15 comprises hydrogel so that a hydrogel body is interposed between said conveying channel 7 and the first surface 15' to allow at least one component 14' to be transferred from said conveying channel 7, through said hydrogel, to said first surface 15' facing the exposed section 2 of the blood vessel 3, e.g. the saphenous vein.

**.** According to an embodiment, the inner volume 6 inside the envelope 4 comprises hydrogel, preferably between and in contact both with said conveying channel 7 and with said first layer 15.

**.** According to an embodiment, said hydrogel is hydrogel that can be photopolymerized, e.g. cross-linked by exposure to ultraviolet radiation.

**.** The first permeable surface 15' can be corrugated to bias the conditioning action either near or in contact with the exposed stretch 2 of the blood vessel 3.

**.** The first layer 15, as well as the inner volume 6, can be made using a variety of materials and methods.

**.** According to an embodiment, said first layer 15 is made of composite material. For example, said composite material of the first layer 15 comprises an essentially porous matrix impregnated with a material permeable to said at least one component 14'. According to an embodiment, said composite material of the first layer 15 comprises a non-woven fabric impregnated with hydrogel.

**.** According to an embodiment, said first layer 15 comprises a semi-permeable membrane. For example, said first layer 15 comprises a selectively permeable membrane.

**.** According to an embodiment, said first layer 15 and/or said inner volume 6 is made of gas-permeable polymeric material, e.g. comprises a layer of silicone material.

**.** According to an embodiment, said first layer 15 and/or said inner volume 6 comprises silicone gel.

**.** According to an embodiment, said first layer 15 and/or said inner volume 6 comprises silk. The term "silk" also means a silk derivative, such as silk fibroin.

**.** According to an embodiment, said first layer 15 and/or said inner volume 6 comprises at least one layer made by electro-spinning. For example, an electro-threaded scaffold can be placed in said inner volume 6.

**.** According to an embodiment, said first layer 15 and/or said inner volume 6 comprises an organized nano-mesh.

**.** According to an embodiment, said first layer 15 and/or said inner volume 6 comprises a non-woven fabric.

**.** According to an embodiment, said conditioning device 1 comprises at least one fluid supply device 18 connected to at least one of said apertures 11, 13 to supply said conveying channel 7 with the working fluid 14. Preferably, said feeding device 18 comprises at least one pump 25 adapted to generate a pressure difference to handle said working fluid 14. According to an embodiment, said supply device 18 comprises at least one reserve tank 27, adapted to contain a predefined volume of working fluid 14.

**.** According to an embodiment, said at least one fluid supply device 18 comprises at least one working fluid conditioning system 21, adapted to regulate the temperature and/or the composition of the working fluid 14, e.g. before it flows in said at least one conveying channel 7.

**.** According to an embodiment, said working fluid conditioning system 21 comprises at least one heat exchanger 31, adapted to regulate the temperature of the working fluid 14. According to an embodiment, said working fluid conditioning system 21 comprises at least one mass exchanger 32, e.g. a gas exchanger. According to an embodiment, said conditioning system of the working fluid 21 comprises at least one delivery and/or collection device 34, e.g. comprising syringe or an auxiliary pumping system, adapted to deliver and/or withdraw a predefined volume of fluid into and/or from the inlet duct 10 of the conditioning device 1. Preferably, said dispensing and/or withdrawal device 34 is adapted to dispense a predetermined volume of active substance into the conveying channel 7, and thus in the inner volume 6 of the envelope 4, and consequently through the first layer 15 onto the exposed stretch 2 of the blood vessel 3.

**.** According to an embodiment, said working fluid conditioning system 21 comprising said heat exchanger 31 and/or said mass exchanger 32 is situated outside the envelope 4, in other words outside the casing 5.

**.** According to an embodiment, said at least one fluid supply device 18 comprises at least one control device 26, e.g. comprises a programmable logic controller PLC, to transmit control signals for automating said fluid supply device 18, e.g. to said pump 25 and/or said working fluid conditioning system 21.

**.** According to an embodiment, a sensing device, e.g. including sensors such as flow meters and/or level sensors and/or temperature sensors and/or humidity sensors and/or concentration sensors of the chemical species of interest, shall be associated with such a control device 26. According to an embodiment, valves 38, e.g. check valves, are provided in said fluid supply device 18 to determine the working fluid path 14.

**.** According to an embodiment, a collection tank 20 is provided to collect the working fluid 14 which flows out of said outlet aperture 13 of the conveying channel 7.

**.** According to a preferred embodiment, said at least one fluid supply device 18 is connected to both said at least one apertures 11,13 of the conveying channel 7, to form a recirculation path 19 for the working fluid 14. A filtering device can be placed in the recirculation path 19.

**.** According to an embodiment, said fluid supply device 18 is integrated inside the body of a handpiece which can be associated with said envelope 4 of the conditioning device 1.

**.** A method of collecting autologous biological material for grafts, e.g. for coronary bypasses, will be described below.

**.** The method of collecting autologous biological material for grafts comprises the step of exposing a stretch 2 of blood vessel 3, e.g. a stretch of saphenous vein.

**.** The method of collecting autologous biological material for grafts also comprises the step of providing a conditioning device 1 according to any of the embodiments described above.

**.** The method of collecting autologous biological material for grafts further comprises the step of making the first surface 15' of the conditioning device 1 face the exposed stretch 2.

**.** The method of collecting autologous biological material for grafts further comprises the step of feeding working fluid 14 into the conveying channel 7 of the conditioning device 1.

**.** The method of collecting autologous biological material for grafts further comprises the step of permeating said first layer 15 with at least one component 14' of the working fluid 14 so that at least one component 14' diffuses to said first surface 15' of the first layer 15.

**.** By virtue of such a method, a conditioning action is applied on said exposed stretch 2 of the blood vessel 3 through said first surface 15' of the first layer 15.

**.** According to a possible mode of operation, the method of collecting autologous biological material for grafts further comprises the step of temporarily wrapping said conditioning device 1 about the exposed stretch 2 of the blood vessel 3. In this manner, a device lumen 17 is formed which acts as a conditioning chamber for the exposed stretch 2 of the saphenous vein 3.

**.** According to a possible mode of operation, the method of collecting autologous biological material for grafts further comprises the step of adjusting the humility in the device lumen 17.

**.** According to a possible mode of operation, the method of collecting autologous biological material for grafts comprises the step of adjusting the acid-base balance, as well as the concentration of oxygen and/or carbon dioxide and/or other substances, in device lumen 17.

**.** According to a possible mode of operation, the method of collecting autologous biological material for grafts comprises the step of administering a pharmacological agent, by means of the delivery of the conditioning device 1 to the exposed stretch 2 of the saphenous vein in said conveying channel 7.

**.** According to a possible mode of operation, the method of collecting autologous biological material for grafts further comprises the step of proceeding with the collection of biological material for autologous grafts from said exposed stretch 2.

**.** By virtue of the features described above, provided either separately or in combination, where applicable, it is possible to respond to the needs mentioned above, and to obtain the listed advantages, in particular:
- a conditioning device 1 is created which has improved control over the conditioning action of the chemical-physical properties of an exposed stretch of saphenous vein, as well as simplified application and increased repeatability compared to known solutions such as traditional surgical gauze;
- at the same time, undesirable mechanical stress on the exposed blood vessel stretch, which causes injuries, is avoided or at least minimized;
- a conditioning device 1 is provided for an exposed stretch of saphenous vein which makes it possible to maintain the physiological conditions, or in any case chemical-physical protective conditions, about the exposed stretch during a procedure of collecting material for autologous grafts, in a repeatable and automated manner;
- at the same time, it makes the conditioning device compatible for use in known standard cardiovascular perfusion and surgical procedures, avoiding the need to impose specific training for the operating room team;
- allows the control of the temperature, humidity, as well as the acid-base balance and concentration of substances interacting with the tissues, about the exposed stretch of saphenous vein or other blood vessel;
- by virtue of the provision of material (e.g. hydrogel) permeable to at least one component 14' of the working fluid, the diffusion of at least one component 14' of the working fluid is allowed from said conveying channel to said first surface facing the exposed stretch, to condition it, while at the same time the working fluid flows into the perfusion channel, applying a convective conveying.

**.** The person skilled in the art may make many changes and adaptations to the embodiments described above, to satisfy contingent needs without however departing from the scope of the appended claims.

### LIST OF REFERENCES

1. Conditioning device
2. Exposed stretch
3. Blood vessel, e.g. saphenous vein
4. Envelope or bag
5. Casing
6. Inner volume or third layer
7. Conveying channel or perfusion duct
8. Locking device or reclosing device
9. Operator
10. Inlet duct
11. First aperture
12. Outlet duct
13. Second aperture
14. Working fluid
14'. Working fluid component
15. First layer
15'. First surface or first face of the first layer
16. Second layer
16'. Second surface or second face of the second layer
17. Device lumen
18. Fluid supply device
19. Recirculation path
20. Collecting tank
21. Working fluid conditioning system
22, 22'. Transverse margin
23, 23', First and second wall of the conveying channel
23". Impermeable wall portion of the conveying channel
24. Free end of the blood vessel
25. Pump
26. Control device
27. Storage tank
28, 28'. Serpentine
29. Union duct
30, 30'. Longitudinal side
31. Heat exchanger
32. Mass exchanger
33. Thickness
34. Dispensing and/or withdrawal device
35. Envelope edge
36. Transverse envelope dimension
37. Patient
38. Valve
39. Cannula
40. Exposed section outer wall
X-X. Longitudinal direction
T-T. Transverse direction

## Claims

1. A conditioning device (1) for conditioning at least one portion of an exposed stretch (2) of a blood vessel (3) for collecting biological material for grafting, comprising:
- an envelope (4);
- at least one conveying channel (7) which extends entirely within the envelope (4);
wherein:
- said envelope (4) comprises a first layer (15) comprising a first surface (15') intended to face said exposed stretch (2), and a second layer (16) comprising a second surface (16'), opposite to said first surface (15);
- said at least one conveying channel (7) comprises at least one first aperture (11) which leads out of the envelope (4), so that said at least one conveying channel (7) can be supplied with working fluid (14) through said at least one first aperture (11);
- said first layer (15) is permeable to at least one component (14') of the working fluid (14), so that, when said working fluid (14) is in said at least one conveying channel (7), said at least one component (14'), permeating said first layer (15) diffuses until it reaches said first surface (15'), to apply a conditioning action on said exposed stretch (2) of the blood vessel (3).

2. A conditioning device (1) according to claim **1**, wherein said second layer (16) is impermeable to said working fluid (14).

3. A conditioning device (1) according to claim **1** or **2,** wherein said first layer (15) forms a barrier for at least one additional component of said working fluid (14), except for said at least one component (14').

4. A conditioning device (1) according to any one of the preceding claims, wherein at least one first wall (23) of the conveying channel (7) is formed by said first layer (15) and at least one second wall (23') of the conveying channel (7) is formed by a third layer (6) formed by the inner volume (6) interposed between said first layer (15) and said second layer (16), said third layer being permeable to said at least one component (14') of the working fluid (14), so as to allow the diffusion of the at least one component (14') through both said first and second walls (23, 23'), distributing itself substantially over the entire first surface (15').

5. A conditioning device (1) according to any one of the preceding claims, further comprising at least one locking device (8), adapted to lock said envelope (4) at least temporarily in a predefined geometric configuration; and wherein said predefined geometric configuration forms a device lumen (17) adapted to receive said at least one exposed stretch (2) of the blood vessel (3).

6. A conditioning device (1) according to claim **5**, wherein said envelope (4) comprises a casing (5) which at least partly delimits said inner volume (6), and wherein said at least one locking device (8) is placed on the margin of said casing (5).

7. A conditioning device (1) according to any one of the preceding claims, wherein said conveying channel (7) defines at least one coil-like perfusion path (28), and preferably at least two opposing coil-like paths (28, 28') which extend along the longitudinal extension of the envelope (4).

8. A conditioning device (1) according to any one of the preceding claims, wherein said at least one conveying channel (7) further comprises at least one second aperture (13) which leads out of the envelope (4); said at least one first aperture (11) acting as an inlet aperture and said at least one second aperture (13) acting as an outlet aperture for said working fluid (14), so that a flow of working fluid (14) passes through said first aperture (11), said conveying channel (7), and said second aperture (13).

9. A conditioning device (1) according to claim **8**, wherein
said at least one first aperture (11) and said at least one second aperture (13) of the conveying channel (7) are placed on the same longitudinal side (30 or 30') of the envelope (4).

10. A conditioning device (1) according to any one of the preceding claims, wherein said first layer (15) comprises hydrogel.

11. A conditioning device (1) according to any one of the preceding claims, wherein said envelope (4) is flexible in at least one transverse direction (T-T), transverse to the longitudinal direction (X-X), so that it can be wrapped about the exposed stretch (2) to embrace the exposed stretch (2) of blood vessel (3).

12. A conditioning device (1) according to any one of the preceding claims, comprising at least one fluid supply device (18) connected to at least one of either said first apertures (11) and at least one second aperture (13) of the conveying channel (7) to supply said conveying channel (7) with the working fluid (14).

13. A conditioning device (1) according to claim **12**, wherein said fluid supply device (18) comprises at least one working fluid conditioning system (21), adapted to regulate a temperature and/or the composition of the working fluid (14), preferably before it passes through said at least one first aperture (11); wherein, preferentially, the working fluid conditioning system (21) comprises at least one heat exchanger (31), adapted to regulate the temperature of the working fluid (14).

14. A conditioning device (1) according to claim **12** or **13,** wherein said at least one fluid supply device (18) is connected to both said at least one first aperture (11) and said at least one second aperture (13) of the conveying channel (7), to form a recirculation path (19) for the working fluid (14).

15. A conditioning device (1) according to claim **12**, **13** or **14**, wherein said at least one fluid supply device (18) comprises at least one control device (26) to transmit control signals for automating said fluid supply device (18).

## Patentansprüche

1. Konditionierungsvorrichtung (1) zum Konditionieren wenigstens eines Teils eines exponierten Abschnitts (2) eines Blutgefäßes (3) zum Sammeln biologischen Materials zum Transplantieren, umfassend:
- eine Hülle (4)
- wenigstens einen Förderkanal (7), welcher sich vollständig innerhalb der Hülle (4) erstreckt;
wobei:
- die Hülle (4) eine erste Schicht (15), welche eine erste Fläche (15'), die dafür gedacht ist, dem exponierten Abschnitt (2) zugewandt zu sein, und eine zweite Schicht (16) umfasst, die eine der ersten Fläche (15) entgegengesetzte zweite Fläche (16') umfasst;
- der wenigstens eine Förderkanal (7) wenigstens eine erste Öffnung (11) umfasst, welche aus der Hülle (4) herausführt, so dass der wenigstens eine Förderkanal (7) durch die wenigstens eine erste Öffnung (11) mit Arbeitsfluid (14) versorgt werden kann;
- die erste Schicht (15) durchlässig für wenigstens eine Komponente (14') des Arbeitsfluids (14) ist, so dass, wenn sich das Arbeitsfluid (14) in dem wenigstens einen Förderkanal (7) befindet, die wenigstens eine Komponente (14'), welche die erste Schicht (15) durchdringt, diffundiert, bis sie die erste Fläche (15') erreicht, um eine Konditionierungswirkung auf den exponierten Abschnitt (2) des Blutgefäßes (3) auszuüben.

2. Konditionierungsvorrichtung (1) nach Anspruch 1, wobei die zweite Schicht (16) undurchlässig für das Arbeitsfluid (14) ist.

3. Konditionierungsvorrichtung (1) nach Anspruch 1 oder 2, wobei die erste Schicht (15) abgesehen von der wenigstens einen Komponente (14') eine Barriere für wenigstens eine zusätzliche Komponente des Arbeitsfluids (14) bildet.

4. Konditionierungsvorrichtung (1) nach einem der vorhergehenden Ansprüche, wobei wenigstens eine erste Wand (23) des Förderkanals (7) durch die erste Schicht (15) gebildet ist und wenigstens eine zweite Wand (23') des Förderkanals (7) durch eine dritte Schicht (6) gebildet ist, welche durch das zwischen der ersten Schicht (15) und der zweiten Schicht (16) eingefügte innere Volumen (6) gebildet ist, wobei die dritte Schicht durchlässig für die wenigstens eine Komponente (14') des Arbeitsfluids (14) ist, um die Diffusion der wenigstens einen Komponente (14') durch sowohl die erste als auch die zweite Wand (23, 23') sich selbst im Wesentlichen über die gesamte erste Fläche (15') verteilend zu ermöglichen.

5. Konditionierungsvorrichtung (1) nach einem der vorhergehenden Ansprüche, ferner umfassend wenigstens eine Verriegelungsvorrichtung (8), welche dazu eingerichtet ist, die Hülle (4) wenigstens vorübergehend in einer vordefinierten geometrischen Konfiguration zu verriegeln; und wobei die vordefinierte geometrische Konfiguration ein Vorrichtungslumen (17) bildet, welches dazu eingerichtet ist, den wenigstens einen exponierten Abschnitt (2) des Blutgefäßes (3) aufzunehmen.

6. Konditionierungsvorrichtung (1) nach Anspruch 5, wobei die Hülle (4) ein Gehäuse (5) umfasst, welches wenigstens teilweise das innere Volumen (6) begrenzt, und wobei die wenigstens eine Verriegelungsvorrichtung (8) an dem Rand des Gehäuses (5) platziert ist.

7. Konditionierungsvorrichtung (1) nach einem der vorhergehenden Ansprüche, wobei der Förderkanal (7) wenigstens einen spulenartigen Perfusionspfad (28) und vorzugsweise wenigstens zwei gegenüberliegende spulenartige Pfade (28, 28') definiert, welche sich entlang der longitudinalen Erstreckung der Hülle (4) erstrecken.

8. Konditionierungsvorrichtung (1) nach einem der vorhergehenden Ansprüche, wobei der wenigstens eine Förderkanal (7) ferner wenigstens eine zweite Öffnung (13) umfasst, welche aus der Hülle (4) herausführt; wobei die wenigstens eine erste Öffnung (11) als eine Einlassöffnung wirkt und die wenigstens eine zweite Öffnung (13) als eine Auslassöffnung für das Arbeitsfluid (14) wirkt, so dass eine Strömung des Arbeitsfluids (14) durch die erste Öffnung (11), den Förderkanal (7) und die zweite Öffnung (13) strömt.

9. Konditionierungsvorrichtung (1) nach Anspruch 8, wobei die wenigstens eine erste Öffnung (11) und die wenigstens eine zweite Öffnung (13) des Förderkanals (7) an der gleichen longitudinalen Seite (30 oder 30') der Hülle (4) platziert sind.

10. Konditionierungsvorrichtung (1) nach einem der vorhergehenden Ansprüche, wobei die erste Schicht (15) Hydrogel umfasst.

11. Konditionierungsvorrichtung (1) nach einem der vorhergehenden Ansprüche, wobei die Hülle (4) in wenigstens einer transversalen Richtung (T-T), transversal zu der longitudinalen Richtung (X-X), flexibel ist, so dass sie um den exponierten Abschnitt (2) gewickelt werden kann, um den exponierten Abschnitt (2) des Blutgefäßes (3) zu umspannen.

12. Konditionierungsvorrichtung (1) nach einem der vorhergehenden Ansprüche, umfassend wenigstens eine Fluidversorgungsvorrichtung (18), welche mit wenigstens einer der ersten Öffnung (11) und wenigstens einer zweiten Öffnung (13) des Förderkanals (7) verbunden ist, um den Förderkanal (7) mit dem Arbeitsfluid (14) zu versorgen.

13. Konditionierungsvorrichtung (1) nach Anspruch 12, wobei die Fluidversorgungsvorrichtung (18) wenigstens ein Arbeitsfluidkonditionierungssystem (21) umfasst, welches dazu eingerichtet ist, eine Temperatur und/oder die Zusammensetzung des Arbeitsfluids (14) zu regulieren, vorzugsweise bevor es durch die wenigstens eine erste Öffnung (11) strömt; wobei bevorzugt das Arbeitsfluidkonditionierungssystem (21) wenigstens einen Wärmetauscher (31) umfasst, welcher dazu eingerichtet ist, die Temperatur des Arbeitsfluids (14) zu regulieren.

14. Konditionierungsvorrichtung (1) nach Anspruch 12 oder 13, wobei die wenigstens eine Fluidversorgungsvorrichtung (18) sowohl mit der wenigstens einen ersten Öffnung (11) als auch der wenigstens einen zweiten Öffnung (13) des Förderkanals (7) verbunden ist, um einen Rückführungspfad für das Arbeitsfluid (14) zu bilden.

15. Konditionierungsvorrichtung (1) nach Anspruch 12, 13 oder 14, wobei die wenigstens eine Fluidversorgungsvorrichtung (18) wenigstens eine Steuervorrichtung (26) umfasst, um Steuersignale zum Automatisieren der Fluidversorgungsvorrichtung (18) zu übertragen.

## Revendications

1. Dispositif de conditionnement (1) pour conditionner au moins une partie d'une section exposée (2) d'un vaisseau sanguin (3) pour collecter un matériau biologique pour une greffe, comprenant :
- une enveloppe (4) ;
- au moins un canal de transport (7) qui s'étend entièrement à l'intérieur de l'enveloppe (4) ;
dans lequel :
- ladite enveloppe (4) comprend une première couche (15) comprenant une première surface (15') destinée à être tournée vers ladite section exposée (2), et une deuxième couche (16) comprenant une deuxième surface (16'), opposée à ladite première surface (15) ;
- ledit au moins un canal de transport (7) comprend au moins une première ouverture (11) qui mène à l'extérieur de l'enveloppe (4), de sorte que ledit au moins un canal de transport (7) peut être alimenté en fluide de travail (14) à travers ladite au moins une première ouverture (11) ;
- ladite première couche (15) est perméable à au moins un composant (14') du fluide de travail (14), de sorte que, lorsque ledit fluide de travail (14) est dans ledit au moins un canal de transport (7), ledit au moins un composant (14') s'infiltrant dans ladite première couche (15) se diffuse jusqu'à ce qu'il atteigne ladite première surface (15') pour appliquer une action de conditionnement sur ladite section exposée (2) du vaisseau sanguin (3).

2. Dispositif de conditionnement (1) selon la revendication 1, dans lequel ladite deuxième couche (16) est imperméable audit fluide de travail (14).

3. Dispositif de conditionnement (1) selon la revendication 1 ou 2, dans lequel ladite première couche (15) forme une barrière pour au moins un composant additionnel dudit fluide de travail (14), sauf pour ledit au moins un composant (14').

4. Dispositif de conditionnement (1) selon l'une quelconque des revendications précédentes, dans lequel au moins une première paroi (23) du canal de transport (7) est formée par ladite première couche (15) et au moins une deuxième paroi (23') du canal de transport (7) est formée par une troisième couche (6) formée par le volume intérieur (6) interposé entre ladite première couche (15) et ladite deuxième couche (16), ladite troisième couche étant perméable audit au moins un composant (14') du fluide de travail (14), de manière à permettre la diffusion dudit au moins un composant (14') à travers à la fois lesdites première et deuxième parois (23, 23'), se distribuant lui-même sensiblement sur toute la première surface (15').

5. Dispositif de conditionnement (1) selon l'une quelconque des revendications précédentes, comprenant en outre au moins un dispositif de verrouillage (8), adapté pour verrouiller ladite enveloppe (4) au moins temporairement dans une configuration géométrique prédéfinie ;
et dans lequel
- ladite configuration géométrique prédéfinie forme une lumière de dispositif (17) adaptée pour recevoir ladite au moins une section exposée (2) du vaisseau sanguin (3).

6. Dispositif de conditionnement (1) selon la revendication 5, dans lequel ladite enveloppe (4) comprend un boîtier (5) qui délimite au moins en partie ledit volume intérieur (6), et dans lequel ledit au moins un dispositif de verrouillage (8) est placé sur la marge dudit boîtier (5).

7. Dispositif de conditionnement (1) selon l'une quelconque des revendications précédentes, dans lequel ledit canal de transport (7) définit au moins un trajet de perfusion du type enroulement (28), et de préférence au moins deux trajets de type enroulement (28, 28') opposés qui s'étendent le long de l'extension longitudinale de l'enveloppe (4).

8. Dispositif de conditionnement (1) selon l'une quelconque des revendications précédentes, dans lequel ledit au moins un canal de transport (7) comprend en outre au moins une deuxième ouverture (13) qui mène à l'extérieur de l'enveloppe (4) ; ladite au moins une première ouverture (11) agissant comme une ouverture d'entrée et ladite au moins une deuxième ouverture (13) agissant comme une ouverture de sortie pour ledit fluide de travail (14), de sorte qu'un flux de fluide de travail (14) passe à travers ladite première ouverture (11), ledit canal de transport (7), et ladite deuxième ouverture (13).

9. Dispositif de conditionnement (1) selon la revendication 8, dans lequel
ladite au moins une première ouverture (11) et ladite au moins une deuxième ouverture (13) du canal de transport (7) sont placées sur le même côté longitudinal (30 ou 30') de l'enveloppe (4).

10. Dispositif de conditionnement (1) selon l'une quelconque des revendications précédentes, dans lequel ladite première couche (15) comprend de l'hydrogel.

11. Dispositif de conditionnement (1) selon l'une quelconque des revendications précédentes, dans lequel ladite enveloppe (4) est flexible dans au moins une direction transversale (T-T), transversale par rapport à la direction longitudinale (X-X), de sorte qu'elle puisse être enveloppée autour de la section exposée (2) pour englober la section exposée (2) du vaisseau sanguin (3).

12. Dispositif de conditionnement (1) selon l'une quelconque des revendications précédentes, comprenant au moins un dispositif d'alimentation en fluide (18) relié à au moins une de l'une ou l'autre desdites premières ouvertures (11) et au moins une deuxième ouverture (13) du canal de transport (7) pour alimenter ledit canal de transport (7) en fluide de travail (14).

13. Dispositif de conditionnement (1) selon la revendication 12, dans lequel ledit dispositif d'alimentation en fluide (18) comprend au moins un système de conditionnement de fluide de travail (21), adapté pour réguler une température et/ou la composition du fluide de travail (14), de préférence avant qu'il passe à travers ladite au moins une première ouverture (11) ; dans lequel, de préférence, le système de conditionnement de fluide de travail (21) comprend au moins un échangeur de chaleur (31), adapté pour réguler la température du fluide de travail (14).

14. Dispositif de conditionnement (1) selon la revendication 12 ou 13, dans lequel ledit au moins un dispositif d'alimentation en fluide (18) est relié à la fois à ladite au moins une première ouverture (11) et à ladite au moins une deuxième ouverture (13) du canal de transport (7), pour former un trajet de recirculation (19) pour le fluide de travail (14).

15. Dispositif de conditionnement (1) selon la revendication 12, 13 ou 14, dans lequel ledit au moins un dispositif d'alimentation en fluide (18) comprend au moins un dispositif de commande (26) pour transmettre des signaux de commande pour automatiser ledit dispositif d'alimentation en fluide (18).
